# FASCICULE DE BREVET EUROPEEN

(11) **EP 3 817 689 B1**
(45) Date de publication et mention de la délivrance du brevet: **27.09.2023**
(21) Numéro de dépôt: 19753415.9
(22) Date de dépôt: 02.07.2019
(51) Int. Cl.: A61F 2/28, A61C 8/00, A61C 8/02

(54) **DISPOSITIF DE CICATRISATION POUR IMPLANT DENTAIRE**
ABUTMENT- UND EINHEILUNGSCUFF FÜR ZAHNIMPLANTAT
ABUTMENT AND HEALING CUFF DEVICE FOR DENTAL IMPLANT

(30) Priorité: 03.07.2018 FR 1856141
(43) Date de publication de la demande: 12.05.2021
(73) Titulaire: Finelle, Gary, 75007 Paris (FR)
(72) Inventeur: Finelle, Gary, 75007 Paris (FR)
(74) Mandataire: Weber, Etienne Nicolas
(86) Numéro de dépôt international: PCT/FR2019/051636
(87) Numéro de publication internationale: WO 2020/008138

(56) Documents cités:
- WO-A1-94/03121
- WO-A1-2012/169667
- WO-A1-2013/109018
- WO-A1-2015/114040
- DE-A1-102014 001 377
- DE-U1- 9 400 537
- US-A- 5 059 123
- US-A- 5 727 945
- US-A1- 2001 055 743
- US-A1- 2009 061 388
- US-A1- 2010 248 187
- US-A1- 2011 171 601
- US-A1- 2014 199 657
- US-A1- 2015 250 592
- US-A1- 2015 305 835
- US-A1- 2017 020 634
- US-A1- 2017 360 565

## Description

L'invention se rapporte au domaine de l'implantologie dentaire.

On connait aujourd'hui des techniques de remplacement dentaire mettant en oeuvre un dispositif implantaire biocompatible. Ce concept biologique repose sur le processus d'ostéointégration d'un implant dans la structure osseuse, sur lequel on vient fixer une prothèse dentaire.

Les protocoles cliniques connus comportent plusieurs étapes espacées dans le temps.

Une première étape consiste à extraire la dent naturelle devant être remplacée. À la suite de cette extraction, on attend durant une période d'au moins plusieurs semaines que la gencive et l'os cicatrisent.

Une deuxième étape, après cicatrisation osseuse et fermeture gingivale, consiste en la pose d'un implant dans la structure osseuse du patient, ce qui implique successivement l'incision de la gencive, une élévation de lambeau et le forage osseux destiné à préparer le lit implantaire. À la suite de la pose de l'implant, un dispositif de protection, qui peut être enfoui (vis de couverture) ou non enfoui (vis ou pilier de cicatrisation), est inséré sur la tête de l'implant. Un tel dispositif de protection permet de maintenir l'ensemble isolé des forces de mastication durant le processus de cicatrisation osseuse appelé ostéointégration. La fermeture du site est obtenue par un système de suture classique permettant de rapprocher les berges gingivales qui s'adaptent autour du pilier de cicatrisation. L'ensemble reste intouché jusqu'à la cicatrisation complète de l'implant dans la structure osseuse par ostéo-intégration et cicatrisation de la gencive autour du pilier de cicatrisation, soit pendant une durée de plusieurs mois.

Une troisième étape consiste à effectuer une prise d'empreinte, c'est-à-dire à enregistrer la position de l'implant et des structures anatomiques environnantes (dents adjacentes, gencives, arcade antagoniste, ...), afin de réaliser la fabrication d'une couronne implantaire anatomique. La prise d'empreinte se fait en retirant le pilier de cicatrisation et en insérant dans l'implant un pilier d'empreinte (également dénommé transfert d'empreinte), qui peut être du type numérique (on parle alors généralement de corps de scannage) ou conventionnel, selon la méthode de prise d'empreinte utilisée par le praticien (prise d'empreinte par scannage numérique en trois dimensions, ou prise d'empreinte manuelle, ou conventionnelle). Après la prise d'empreinte, le pilier de cicatrisation est revissé à l'implant durant le temps de fabrication de la prothèse, dans l'attente de l'étape de pose de celle-ci.

La quatrième et dernière étape est celle de la pose de la prothèse dentaire, qui est réalisée en vissant une structure implanto-prothétique en une partie (dans le cas d'une réhabilitation transvissée) ou en deux parties (dans le cas d'une réhabilitation scellée).

Les protocoles conventionnels, tels que celui décrit ci-dessus, présentent l'inconvénient de multiplier les interventions sur le patient, et notamment de nécessiter plusieurs interventions sur la gencive après sa cicatrisation suite à la pose de l'implant. En outre, l'extraction de la dent naturelle et la pose de l'implant sont généralement réalisées lors de deux interventions indépendantes et espacées de plusieurs mois, afin de permettre au site osseux de se régénérer, aussi bien au niveau osseux que muqueux. Ces différentes interventions impliquent une prise en charge chirurgicale d'une durée incompressible et relativement importante, s'étalant sur plusieurs mois. D'autre part, chaque procédure présente un risque opératoire lié au geste chirurgical lui-même. Ainsi, la multiplicité des interventions augmente l'invasivité du traitement pour le patient ainsi que les risques de suites opératoires.

Afin de limiter le nombre d'interventions, il a été développé des techniques consistant à effectuer les étapes d'extraction de la dent et de pose de l'implant lors d'une même intervention. Chacun des documents US 2001 /055743 A1, US 2009/061388 A1 et WO 2012/169667 A1 divulguent un pilier intermédiaire dentaire, destiné à être inséré dans un implant dentaire, le pilier intermédiaire comprenant d'une part un dispositif de connexion d'un élément de fermeture de forme anatomique pour la fermeture du site alvéolaire et d'autre part une partie proximale pour la fixation d'un élément prothétique dentaire.

Toutefois, la morphologie de chaque alvéole d'extraction étant spécifique, et les dimensions du site chirurgical étant plus importantes que les vis de cicatrisation industrialisées, l'obtention de la fermeture du site opératoire contraint les cliniciens à effectuer des manipulations chirurgicales invasives, ainsi que des déplacements de lambeau importants, ce qui génère des phénomènes de résorptions tissulaires et des suites opératoires souvent douloureuses.

Il existe donc un besoin de mettre en oeuvre des protocoles pouvant simplifier la prise en charge, réduire l'invasivité du traitement et limiter les interventions sur le patient depuis l'extraction de la dent jusqu'à la fixation de la prothèse implantaire définitive.

Ainsi, la présente invention a pour but de remédier aux inconvénients de l'état de la technique, et plus particulièrement ceux ci-dessus exposés, en proposant un dispositif de cicatrisation péri-implantaire qui permette :
- d'améliorer la cicatrisation de la gencive en empêchant l'affaissement gingival lié à l'absence de soutien mécanique (après extraction) ;
- de stabiliser le caillot sanguin dans un espace clos favorable à la régénération osseuse ;
- de limiter le nombre d'interventions sur le patient ;
- de limiter la durée du traitement ;
- de limiter les manipulations sur l'implant et/ou les éléments solidarisés à l'implant.

À cet effet, l'invention concerne un pilier intermédiaire dentaire, destiné à être inséré dans un implant dentaire, le pilier intermédiaire comprenant :
- une partie inférieure, destinée à être insérée dans un implant ;
- une partie intermédiaire, comportant un dispositif externe de connexion d'un élément de fermeture de forme anatomique pour la fermeture du site alvéolaire post-extractionnel ;
- une partie supérieure, comportant un dispositif de fixation d'un organe, tel qu'un pilier d'empreinte ou un pilier prothétique.

Ainsi, en fournissant un pilier intermédiaire permettant la fixation d'éléments de fermeture anatomiques de formes et de dimensions variées, personnalisables le cas échéant, l'invention permet d'assurer la cicatrisation du site chirurgical post-extractionnel en reproduisant fidèlement la morphologie de l'émergence de la dent naturelle initialement présente. Le pilier intermédiaire conforme à l'invention permet en effet de connecter, au niveau de la partie intermédiaire du pilier (qui correspond à la partie transmuqueuse du pilier, c'est-à-dire la partie du pilier qui se trouve dans la zone gingivale du site chirurgical quand le pilier est positionné sur un implant inséré dans la structure osseuse), un élément de fermeture anatomique biocompatible pour assurer la fermeture du site alvéolaire post-extractionnel (c'est-à-dire l'alvéole formée par le contour gingival immédiatement après extraction de la dent d'origine). Cet élément peut ainsi être choisi dans un ensemble couvrant les différentes morphologies cervicales des différentes dents de l'arcade dentaire, rendant ainsi le dispositif de cicatrisation (formé par l'ensemble du pilier intermédiaire et d'un élément de fermeture conformes à l'invention) totalement adaptable durant l'intervention et personnalisable à une forme parfaitement anatomique, forme déterminée par le contour gingival immédiatement après extraction.

Si nécessaire, une adaptation finale de l'élément de fermeture, se fera, *in vivo,* sur le site chirurgical à l'aide d'une résine composite de restauration (par exemple une résine photopolymérisable). Si nécessaire également, l'élément de fermeture pourra être retaillé pour une adaptation très fine par rapport au site chirurgical (en étant par exemple coupé aux ciseaux ou fraisé à la fraise par le chirurgien-dentiste).

Le pilier intermédiaire conforme à l'invention présente en outre un dispositif de connexion sur sa partie occlusale (ou supérieure) permettant la fixation au-dessus de l'élément de fermeture anatomique d'un élément tel qu'un pilier d'empreinte ou un élément prothétique (dent provisoire par exemple). Ainsi, il n'est nécessaire de retirer ni l'élément de fermeture anatomique ni le pilier intermédiaire pour effectuer la prise d'empreinte en vue de la fabrication d'une prothèse. On limite ainsi le nombre d'éléments devant être successivement connectés à l'implant. Enfin, le pilier conforme à l'invention peut également servir de pilier intermédiaire en supportant le pilier prothétique final. Dans une telle configuration, le pilier conforme à l'invention n'est jamais retiré après sa pose initiale, ce qui limite les interventions sur le patient et diminue ainsi les risques et traumatismes liées aux interventions, notamment vis-à-vis de la lésion de l'attache biologique entre la muqueuse péri-implantaire et le pilier prothétique.

La disposition externe du dispositif de connexion de l'élément de fermeture permet de rendre ce dispositif de connexion accessible depuis la surface latérale (extérieure) du pilier, pour une fixation externe (ou latérale) de l'élément de fermeture sur le pilier. Par fixation externe, ou latérale, on entend notamment que le dispositif de fixation est accessible même lorsque le pilier intermédiaire est connecté à un implant. Ainsi, l'élément de fermeture peut être fixé au pilier intermédiaire (et, inversement, retiré de ce dernier) alors que le pilier intermédiaire est déjà fixé à un implant préalablement inséré dans la structure osseuse du patient. Le pilier intermédiaire est donc configuré pour que le dispositif externe de connexion d'un élément de fermeture de forme anatomique reste accessible lorsque le pilier est fixé à un implant. En outre, le pilier intermédiaire est configuré pour que le dispositif de fixation présent sur sa partie supérieure reste accessible même lorsqu'un élément de fermeture est attaché au pilier intermédiaire.

Avantageusement, l'élément de fermeture est fixé au dispositif externe de connexion du pilier intermédiaire par coopération de forme, et notamment par emboîtement élastique ou clipsage. L'élément de fermeture peut ainsi être facilement lié au pilier intermédiaire *in vivo,* de manière fiable, sans nécessiter d'éléments de fixation tel qu'une vis.

Dans une réalisation, le dispositif externe de connexion est configuré pour permettre la fixation d'un élément de fermeture par coopération de forme, notamment par clipsage.

Dans une réalisation, le dispositif externe de connexion d'un élément de fermeture comporte au moins une gorge de forme générale circulaire ménagée sur la surface externe de la partie intermédiaire du pilier intermédiaire.

Dans une réalisation, le pilier intermédiaire comporte au moins deux gorges superposées.

Dans une réalisation, le pilier intermédiaire comporte au moins trois gorges superposées.

Dans une réalisation, la ou les gorge(s), est (sont) continue(s) sur tout le pourtour de la partie intermédiaire du pilier intermédiaire.

Dans une réalisation, la ou les gorge(s) est (sont) discontinue(s) sur le pourtour de la partie intermédiaire du pilier intermédiaire, et comportent une pluralité de secteurs adjacents, par exemple deux, quatre, six ou huit secteurs adjacents.

Dans une réalisation, le pilier intermédiaire est configuré pour que la partie intermédiaire soit, lorsque celui-ci est connecté à un implant posé dans la structure osseuse d'un patient, située dans la zone gingivale du site chirurgical, c'est-à-dire la partie transmuqueuse.

Dans une réalisation, le dispositif de fixation d'un organe comporte un taraudage.

Dans une réalisation, le dispositif de fixation d'un organe est associé à un dispositif d'indexation de la position de l'organe par rapport au pilier intermédiaire, et notamment de la position angulaire de l'organe.

Dans une réalisation, le pilier intermédiaire comporte une vis permettant la fixation du pilier intermédiaire dans un implant, notamment une vis prisonnière.

Dans une réalisation, le pilier intermédiaire comporte un alésage traversant, une première portion de l'alésage étant configurée pour permettre le passage de la vis, une deuxième portion de l'alésage comportant le dispositif de fixation d'un organe.

L'invention concerne également un dispositif de cicatrisation comportant un pilier intermédiaire tel que défini ci-dessus et un élément de fermeture anatomique, l'élément de fermeture comportant une partie de fixation destinée à coopérer avec le dispositif externe de connexion du pilier intermédiaire, et une partie de fermeture présentant une forme anatomique.

Dans une réalisation, la partie de fixation de l'élément de fermeture comporte une ou plusieurs languettes déformables élastiquement et configurées pour coopérer avec le dispositif externe de connexion du pilier intermédiaire.

Dans une réalisation, l'élément de fermeture comporte un matériau biocompatible, notamment un polymère biocompatible, tel que le polyétheréthercétone (ou PEEK) ou le polyuréthane.

Dans une réalisation, la partie de fermeture présente une bordure extérieure de forme dentelée et/ou comportant une surface présentant une rugosité favorisant l'adhésion d'une résine, notamment une résine composite photopolymérisable.

L'invention concerne également un ensemble d'un pilier intermédiaire tel que défini plus haut et d'un pilier prothétique pour une prothèse implantaire, le pilier prothétique étant connecté à au pilier intermédiaire, le pilier prothétique comportant à cet effet un tronçon tubulaire conformé pour entourer au moins partiellement la partie intermédiaire du pilier intermédiaire lorsque le pilier prothétique et le pilier intermédiaire sont connectés par l'intermédiaire du dispositif de fixation du pilier intermédiaire.

L'invention concerne également un kit d'aménagement gingival supra-implantaire comportant un pilier intermédiaire tel que défini plus haut et plusieurs éléments de fermeture tels que défini ci-avant.

L'invention concerne également un outil pour la sélection d'un élément de fermeture tel que défini plus haut, caractérisé en ce que l'outil comporte un ou plusieurs gabarits reproduisant le profil en deux dimensions d'un élément de fermeture (ou de l'ensemble formé par un pilier intermédiaire et un élément de fermeture), ou la forme en trois dimensions d'un élément de fermeture (ou de l'ensemble d'un pilier intermédiaire et d'un élément de fermeture).

La présente invention sera mieux comprise à la lecture de la description détaillée qui suit, faite en référence aux dessins annexés, dans lesquels :
- les figures 1a et 1b représentent un implant, vu respectivement en perspective et en coupe ;
- les figures 2a et 2b représentent un pilier intermédiaire conforme à l'invention, vu respectivement en perspective et en coupe ;
- la figure 3 est une vue en coupe du piler intermédiaire des figures 2a et 2b équipé d'un élément de fermeture conforme à l'invention ;
- les figures 4a et 4b sont des vues en perspective d'un deuxième et d'un troisième exemple de réalisation d'un pilier intermédiaire conforme à l'invention ;
- les figures 5a à 7f illustrent différentes formes d'un élément de fermeture conforme à l'invention, selon les différentes anatomies dentaires ;
- les figures 8a à 8d illustrent différentes étapes de la pose d'une prothèse dentaire à l'aide d'un pilier intermédiaire conforme à l'invention ;
- la figure 9 est une vue en coupe de la gencive d'un patient, dans laquelle une prothèse dentaire est fixée à un pilier intermédiaire conforme à l'invention ;
- les figures 10 et 11 illustrent deux outils permettant de faciliter l'utilisation d'un pilier intermédiaire conforme à l'invention.

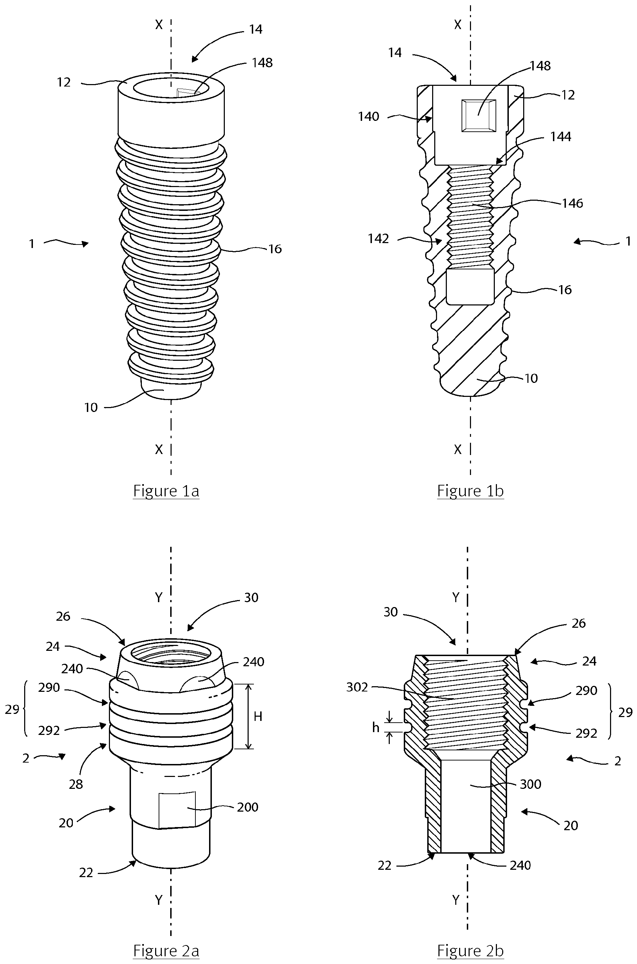

Les figures 1a et 1b représentent un exemple d'implant dentaire, respectivement vu en perspective et vu en coupe. L'implant 1 représenté sur les figures 1a et 1b est, de manière connue, configuré pour être posé et stabilisé mécaniquement par technique de vissage dans l'os maxillaire ou mandibulaire d'un patient. Après un temps de cicatrisation de plusieurs semaines, la stabilité mécanique est remplacée par une stabilité biologique plus fiable, l'implant est alors ostéo-intégré, et la pose de la prothèse définitive peut alors être envisagée. L'implant 1 est dans l'exemple de forme générale tronconique d'axe X (mais peut bien entendu être de toute autre forme adaptée, par exemple cylindrique). L'implant 1 comporte une première extrémité 10, ou extrémité apicale, qui est fermée, et une deuxième extrémité 12, ou extrémité cervicale, opposée à la première extrémité, et au niveau de laquelle débouche un alésage 14 borgne pratiqué dans le corps de l'implant 1. L'implant 1 comporte un filetage externe 16, pouvant être un filetage de type auto-taraudant ou non. L'alésage 14 comprend une première portion 140, non filetée. La première portion est reliée, par l'intermédiaire d'un épaulement 144 à une deuxième portion 142, qui comporte un taraudage 146. La première portion 140 de l'alésage 14 comporte au moins un élément d'indexation interne 148, destinée à coopérer avec une forme correspondante prévue sur un élément devant être connecté à l'implant 1, tel qu'un pilier intermédiaire. Dans l'exemple, l'élément d'indexation 148 forme une protubérance depuis la surface de la première portion 140 de l'alésage 14.

Les figures 2a et 2b représentent un pilier intermédiaire 2 conforme à l'invention, vu respectivement en perspective et en coupe. Le pilier intermédiaire 2 est réalisé dans tout matériau biocompatible adapté, tel que le titane, le polyétheréthercétone (ou PEEK), ou un matériau céramique comme par exemple la zircone. Le pilier intermédiaire 2 présente une forme générale qui est tubulaire et globalement symétrique de révolution d'axe Y. Le pilier intermédiaire 2 présente une première partie 20 (ou partie inférieure 20), dont une extrémité libre forme l'extrémité inférieure 22 du pilier intermédiaire, la première partie 20 étant conformée pour être insérée dans un implant dentaire tel que l'implant 1 des figures 1a et 1b. L'extrémité opposée du pilier intermédiaire, ou extrémité supérieure 26, est constituée par l'extrémité libre d'une deuxième partie 24, ou partie supérieure 24 du pilier intermédiaire 2.

Entre les parties inférieure 20 et supérieure 24 est disposée une partie intermédiaire 28. Le pilier intermédiaire 2 est configuré pour que la partie intermédiaire 28 corresponde à la partie transmuqueuse du pilier, c'est-à-dire que lorsque le pilier intermédiaire 2 est positionné sur un implant posé dans la structure osseuse d'un patient, alors la partie intermédiaire 28 se trouve dans la zone gingivale du site chirurgical.

Le pilier intermédiaire 2 comporte un alésage 30 traversant, débouchant au niveau des extrémités supérieure 26 et inférieure 22 du pilier intermédiaire. L'alésage 30 constitue un puits d'accès de vis.

L'alésage 30 comporte une première portion 300, débouchant sur un orifice 220 situé au niveau de l'extrémité inférieure 22 du pilier intermédiaire 2. La première portion 300 est destinée à permettre le passage d'une vis de fixation assurant la fixation du pilier intermédiaire à l'implant 1.

La première portion 300 de l'alésage 30 est surmontée par une deuxième portion 302, de diamètre interne supérieur au diamètre interne de la première portion 300. La deuxième portion 302 comporte un filetage 304 interne permettant la fixation d'un organe externe, notamment au moyen d'une vis.

Sur la surface extérieure de la partie inférieure 20, le pilier intermédiaire 2 comporte un élément d'indexation 200, dont la forme est complémentaire de celle de l'élément d'indexation 148 de l'implant 1, permettant ainsi un positionnement angulaire indexé du pilier intermédiaire 2 dans l'implant 1.

La partie intermédiaire 28 du pilier intermédiaire 2 est dans l'exemple de forme générale cylindrique d'axe Y. Conformément à l'invention, la partie intermédiaire 28 comporte au moins un dispositif externe de connexion 29 d'un élément de fermeture anatomique. Dans l'exemple, la partie intermédiaire comporte un dispositif externe de connexion 29 à deux niveaux (ou étages), sous la forme de deux gorges 290, 292 circulaires superposées, soit une gorge supérieure 290 et une gorge inférieure 292.

Comme mentionné plus haut, la partie intermédiaire 28 est disposée entre les parties inférieure 20 et supérieure 24 du pilier intermédiaire 2. Ainsi la partie intermédiaire 28 est surmontée par la partie supérieure 24, qui est dans l'exemple de forme générale tronconique d'axe Y. Sur la surface extérieure de la partie supérieure 24, le pilier intermédiaire 2 comporte une pluralité d'éléments d'indexation externes 240, qui sont dans l'exemple des méplats 240, permettant l'indexation d'un élément fixé au pilier intermédiaire 2. Dans l'exemple, il est prévu quatre méplats répartis régulièrement sur le pourtour de la partie supérieure 24, mais on pourra prévoir alternativement un nombre différent d'éléments d'indexation, par exemple six ou huit. On pourra également prévoir, alternativement ou en complément, un ou plusieurs éléments d'indexation internes, disposés au sein de l'alésage 30.

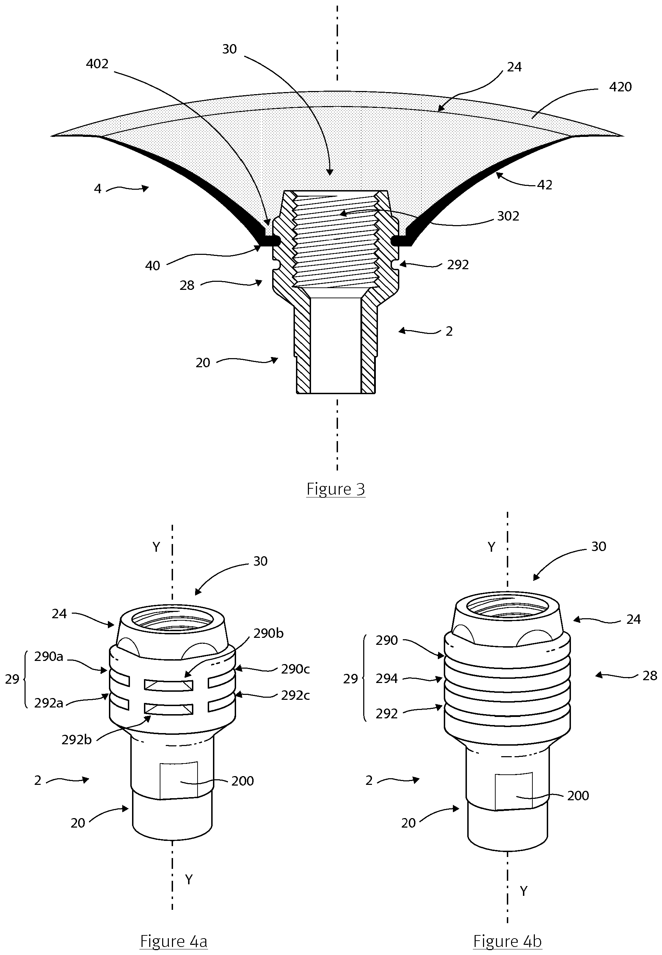

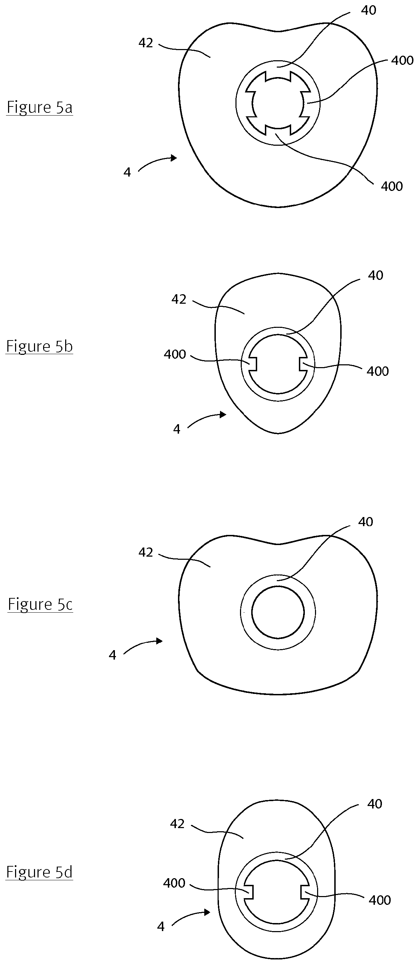

Comme montré sur la figure 3, grâce aux gorges 290, 292 ménagées dans la surface extérieure de la partie intermédiaire 28 du pilier intermédiaire 2, un élément de fermeture 4 anatomique peut être fixé simplement et facilement, de manière fiable, au pilier intermédiaire 2 par coopération de forme, par exemple par clipsage. L'élément de fermeture 4 comporte à cet effet une partie de fixation 40 de forme générale annulaire, la partie de fixation 40 étant apte à coopérer par coopération de forme, notamment par clipsage, avec l'une des gorges 290, 292 du pilier intermédiaire 2. Dans l'exemple, la partie de fixation 40 comporte à cet effet une ou plusieurs languettes 400 (visibles notamment sur les figures 5a à 5d) orientées radialement, déformables élastiquement, et aptes à être insérées dans l'une des gorges 290, 292 du pilier intermédiaire 2 pour assurer la retenue de l'élément de fermeture 4 par emboîtage élastique et/ou clipsage. Avantageusement, la partie de fixation 40 comporte une partie tubulaire définissant un espace annulaire 402 entre la partie de fixation 40 et le pilier intermédiaire 2. Ainsi, cet espace annulaire 402 pourra si nécessaire être comblé avec de la résine afin de sécuriser la fixation de l'élément de fermeture 4 sur le pilier intermédiaire 2. La partie de fixation 40 est reliée à une partie de fermeture 42 anatomique, dont la forme et les dimensions peuvent être adaptées finement à l'anatomie du patient qui doit recevoir le dispositif de cicatrisation formé par le pilier intermédiaire 2 et l'élément de fermeture 4. La partie de fermeture 42 présente avantageusement une forme générale en collerette, et présente notamment une forme évasée depuis la partie de fixation 40 jusqu'à une bordure extérieure 420. Dans l'exemple, la bordure extérieure 420 de la partie de fermeture 42 présente une forme sensiblement plate. Avantageusement, la bordure extérieure 420 présentera une surface de forte rugosité (par exemple une surface microsablée), de manière à favoriser l'adhésion de la résine. Alternativement ou en complément, on pourra prévoir que la bordure extérieure 420 présente une forme dentelée ou crénelée, afin de permettre une adhésion améliorée de la résine biocompatible qui sera déposée au niveau de cette bordure extérieure, comme on le verra plus bas. L'élément de fermeture 4 sera réalisé dans tout matériau biocompatible adapté, comme par exemple le PEEK ou un polymère biocompatible tel que le polyuréthane. L'épaisseur de la partie de fixation 40 sera notamment comprise entre 0,4 et 1,5 millimètres, et par exemple égale à 0,5 millimètres. L'épaisseur de la partie de fermeture 42 pourra par exemple être comprise entre 0,5 et 4 millimètres. La bordure extérieure 420 de la partie de fermeture sera par exemple comprise entre 0,5 et 2,5 millimètres.

À titre d'exemple, le diamètre externe de la partie intermédiaire 28 du pilier 2 pourra être comprise entre 3 et 6 millimètres, et dépendra notamment du diamètre de l'implant. La hauteur H de la partie intermédiaire pourra être comprise entre 1 et 5 millimètres. La hauteur des gorges 290, 292 pourra par exemple être comprise entre 0,4 et 1,5 millimètres, et par exemple égale à 0,5 millimètre. Dans le cas d'un pilier comportant plusieurs étages de fixation, les gorges seront par exemple espacées d'une valeur h comprise entre 0,5 et 1,5 millimètre, et par exemple égale à 1 millimètre.

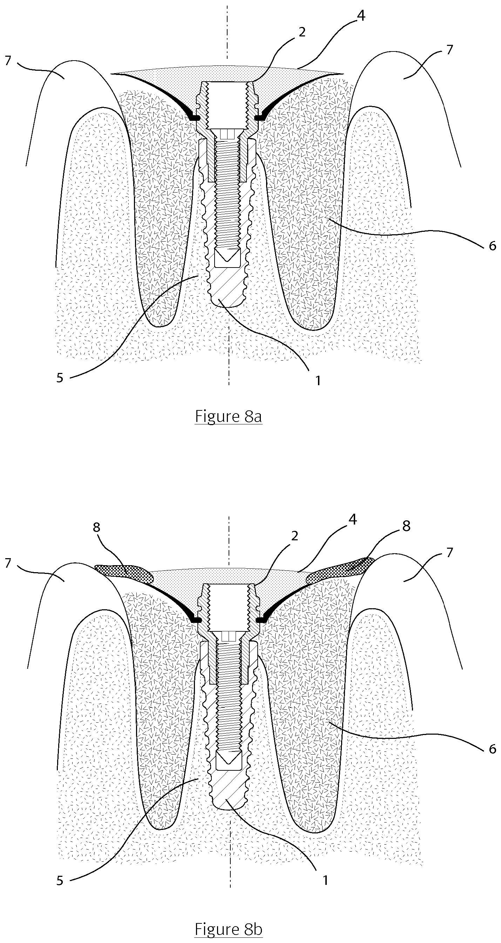

On décrit ci-après les principales étapes, en relation notamment avec les figures 8a à 8d, de l'utilisation d'un pilier intermédiaire 2 et d'un élément de fermeture 4 conformes à l'invention.

La figure 8a montre la gencive d'un patient vue en coupe, dans laquelle, on a successivement :
- inséré dans la structure osseuse 5 l'implant 1 ;
- utilisé un matériau de substitut osseux 6 pour combler l'alvéole post-extractionnelle, afin de préserver le volume osseux et empêcher la résorption durant les périodes de cicatrisation ;
- sélectionné un pilier intermédiaire 2 conforme à l'invention de taille adaptée, puis inséré et solidarisé ce dernier à l'implant 1 au moyen d'une vis de fixation 3. Le pilier intermédiaire sera notamment choisi de sorte qu'une fois fixé à l'implant, la partie intermédiaire 28 se situe dans la partie transmuqueuse du patient ;
- sélectionné et positionné sur le pilier intermédiaire 2 un élément de fermeture 4 dont la forme, la taille et la hauteur de positionnement (dans le cas où le pilier intermédiaire 2 comporte un dispositif de connexion externe à plusieurs niveaux) sont choisis afin de correspondre au plus proche de l'anatomie de l'émergence alvéolaire du patient. En particulier, on observe que l'élément de fermeture 4 présente une forme et une hauteur (c'est-à-dire la dimension selon l'axe de révolution Y du pilier intermédiaire) permettant au bord extérieur de la partie de fermeture 42 de venir à proximité de l'ouverture de la gencive marginale 7 du patient. Ainsi, après la procédure d'extraction, d'implantation, et de mise en place du pilier intermédiaire, l'élément de fermeture 4 permet d'obturer partiellement le site alvéolaire, ne laissant dans un premier temps qu'un faible espace entre le pilier et la gencive marginale 7 (par exemple un espace d'un à deux millimètres). Bien entendu, l'élément de fermeture 4 pourra le cas échéant être retaillé par le praticien de manière à le rendre légèrement sous dimensionné par rapport aux dimensions de l'alvéole.

La figure 8b montre l'étape finale de fermeture de la gencive après positionnement du pilier intermédiaire 2 et de l'élément de fermeture 4. Cette étape consiste à fermer le site alvéolaire, par le biais d'un apport d'un matériau biocompatible 8 de type résine composite (notamment une résine photopolymérisable), permettant de créer une barrière hermétique entre le site chirurgical et la cavité orale, réduisant ainsi le risque de contamination et de passage d'éléments exogènes indésirables (nourriture par exemple). Le dispositif de cicatrisation conforme à l'invention assure un rôle important dans le soutien mécanique de l'architecture gingivale post-extractionnelle et dans l'aménagement gingival transmuqueux. Il joue également un rôle dans la stabilité du caillot sanguin et la réponse biologique associée au processus de régénération osseuse.

On notera que le praticien équipé d'un système de fabrication assistée par ordinateur pourra avantageusement réaliser sur place un élément de fermeture adapté, après prise d'empreinte du site chirurgical du patient. La fabrication pourra alors être réalisée par des techniques de fabrication additive ou soustractive.

Comme visible sur les figure 8a à 8d, on notera que le positionnement externe de l'élément de fermeture 4 permet une fixation simple, sans éléments supplémentaires (tels qu'une vis).

On comprend donc que l'invention permet de simplifier grandement la mise en oeuvre des protocoles d'extraction/implantation immédiate, en proposant une solution de fermeture du site alvéolaire parfaitement adaptée à chaque patient. En effet, le pilier intermédiaire 2 permet une parfaite individualisation du dispositif de cicatrisation, adaptée à chaque situation clinique une fois la dent extraite. Ainsi, l'extraction de la dent, les étapes de forage, de pose d'implant, de pose du pilier intermédiaire 2, de l'élément de fermeture 4, et, le cas échéant, d'une couche de résine sont réalisées en une seule intervention.

Avantageusement, le pilier intermédiaire conforme à l'invention est configuré pour ne pas émerger ou émerger faiblement au-delà de la zone cervicale, afin d'éviter les sollicitations mécaniques inhérentes aux fonctions de l'appareil manducatoire (mastication, déglutition...). Une fois fixé fermement, le dispositif de cicatrisation sera laissé intouché, pour permettre une cicatrisation normale jusqu'à l'obtention de l'ostéo-intégration. Cette étape prend généralement quelques mois.

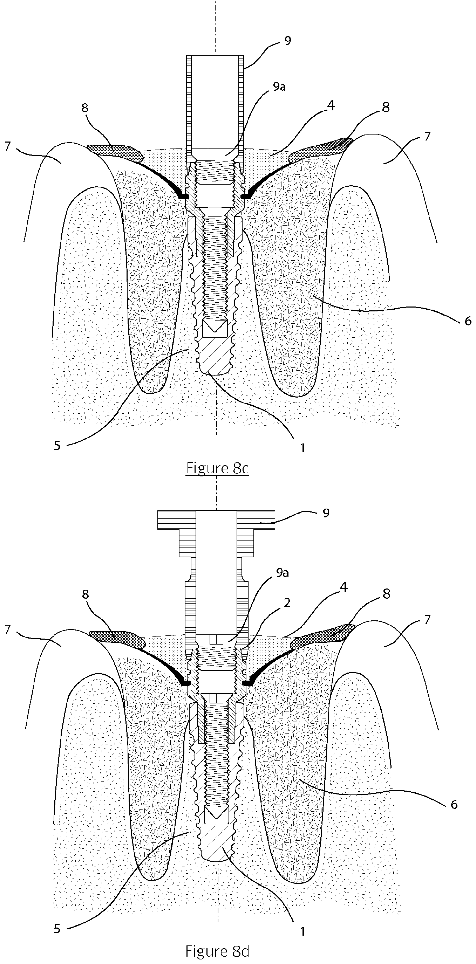

Une fois l'étape de cicatrisation achevée, il est nécessaire de prendre une empreinte de la position tridimensionnelle (incluant l'indexation) de l'implant en relation avec les structures anatomiques et dentaire du patient (ce qui inclut la position de la gencive telle que stabilisée autour du dispositif de cicatrisation). Cette empreinte permettra de générer un modèle de travail à partir duquel sera réalisée la prothèse. Conformément à l'invention, le pilier intermédiaire 2 est configuré pour que l'étape de prise d'empreinte puisse être réalisée sans démontage du pilier intermédiaire 2 (et sans retirer l'élément de fermeture anatomique 4), contrairement aux procédés connus mettant en oeuvre des vis de cicatrisation conventionnelles (ces vis de cicatrisation devant impérativement être retirées pour laisser place à un pilier d'empreinte). Comme visible sur les figures 8c et 8d, qui illustrent respectivement une prise d'empreinte numérique et une prise d'empreinte conventionnelle, ni le démontage du pilier intermédiaire 2 ni celui de l'élément de fermeture 4 ne sont requis pour connecter un pilier d'empreinte, puisque la partie supérieure 24 du pilier intermédiaire, incluant le dispositif de fixation 304 reste accessible même en présence d'un élément de fermeture 4 anatomique. La fixation d'un pilier d'empreinte 9, de type numérique (figure 8c) ou conventionnel (figure 8d) est réalisée au moyen d'une vis de fixation 9a, qui coopère avec le filetage 304 interne de l'alésage 30 du pilier intermédiaire 2. En outre, les éléments d'indexation 240 coopèrent avec des éléments d'indexation de forme complémentaire présents sur le pilier d'empreinte 9, et permettant un positionnement angulaire indexé de ce dernier.

Une fois la prise d'empreinte réalisée, une étape consiste à fabriquer la prothèse dentaire en utilisant les données recueillies lors de l'étape de prise d'empreinte.

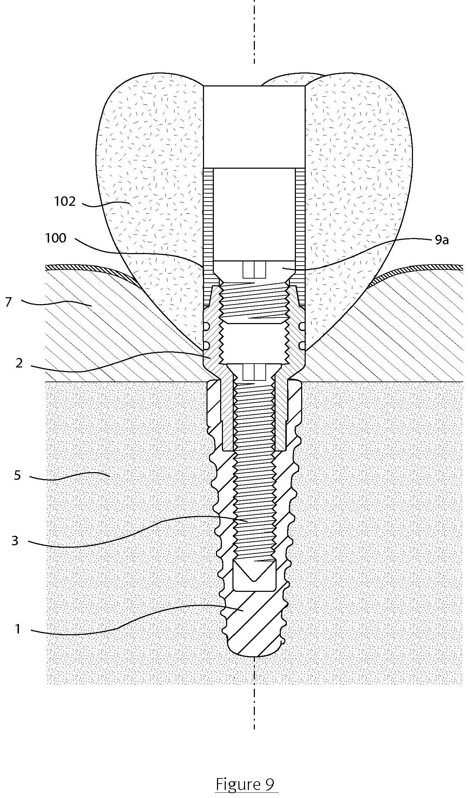

Une étape finale consiste à installer la prothèse dentaire, visible sur la figure 9. La prothèse dentaire peut être fabriquée afin d'être vissée directement à la plateforme implantaire, dans ce cas l'ensemble de cicatrisation formé par le pilier intermédiaire 2 et l'élément de fermeture 4 est déposé le jour de l'assemblage prothétique final. Dans l'exemple de la figure 9, il a été envisagé de conserver le pilier intermédiaire 2, qui constitue alors un pilier intermédiaire pour la fixation d'un pilier prothétique 100. La prothèse dentaire 102 est fixée à l'aide du pilier prothétique 100, ce dernier étant fixé dans le pilier intermédiaire 2, au moyen d'une vis de fixation 9a qui coopère avec le filetage interne 304, après retrait de l'élément de fermeture 4. Cette configuration présente notamment l'avantage de ne jamais nécessiter le retrait du pilier intermédiaire 2, depuis son insertion initiale dans l'implant 1. Cela permet de réduire encore davantage les manipulations nécessaires au cours de la restauration dentaire. Dans l'exemple, la prothèse dentaire 102 est conformée pour entourer au moins partiellement la partie intermédiaire 28 du pilier intermédiaire 2, et recouvre le dispositif externe de connexion 29. Avantageusement, le pilier prothétique 100 peut comporter un tronçon tubulaire (non représenté) se prolongeant de manière à entourer au moins partiellement la partie intermédiaire 28, de préférence intégralement. Dans une telle configuration, le tronçon tubulaire entoure au moins le dispositif externe de connexion 29.

Les figures 5a à 7f montrent différentes formes de l'élément de fermeture 4.

Comme visible sur les figures 5a, 5b, 5c et 5d, la forme de l'élément de fermeture 4 est avantageusement adaptée au type de la dent qui doit être remplacée. On prévoira donc avantageusement au moins quatre formes différentes de l'élément de fermeture 4 : une première forme correspondant à une molaire maxillaire (figure 5a), une deuxième forme correspondant à une molaire mandibulaire (figure 5b), une troisième forme correspondant à une prémolaire (figure 5c) et une quatrième forme correspondant à une incisive (figure 5d).

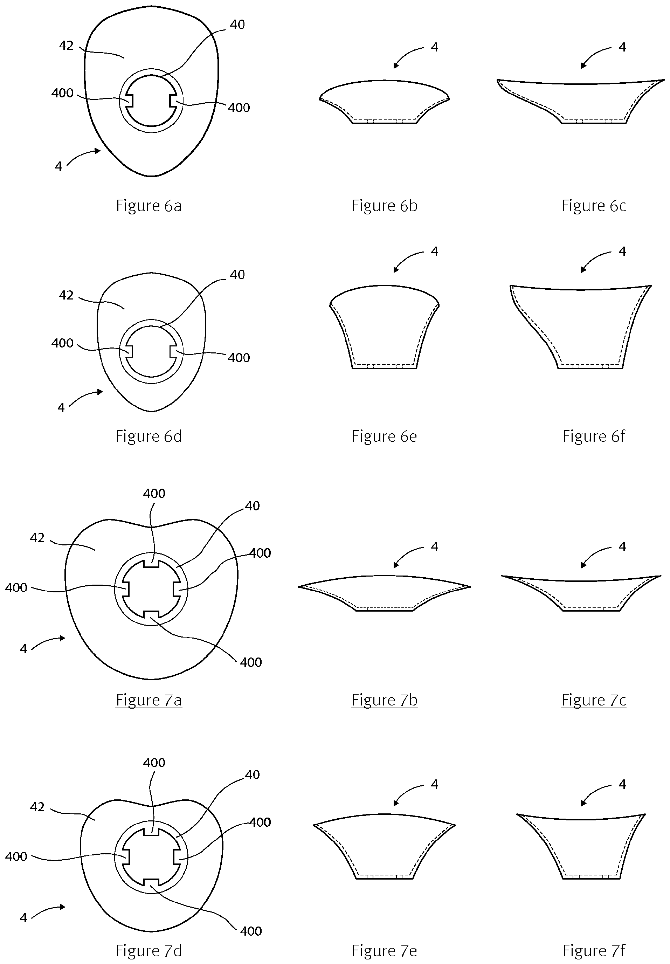

Avantageusement, pour chacune des formes différentes, on pourra prévoir plusieurs tailles différentes. Par exemple, les figures 6a et 6d montrent deux exemples de tailles différentes de la première forme (correspondant à une molaire maxillaire), tandis que les figures 7a et 7d montrent deux exemples de tailles différentes pour la quatrième forme (correspondant à une incisive).

Avantageusement encore, on prévoira, pour chaque taille et chaque forme, des hauteurs et profils différents, comme montré sur les figures 6b, 6c, 6e et 6f pour la première forme, et sur les figures 7b, 7c, 7e et 7f pour la quatrième forme.

Les figures 3a et 3b montrent deux exemples de réalisation du pilier intermédiaire conforme à l'invention.

Dans l'exemple de la figure 3a, les gorges 290, 292 sont discontinues, étant ainsi formées par une pluralité de secteurs adjacents 290a, 290b, 290c et 292a, 292b, 292c. Dans l'exemple, chaque gorge est formée de quatre secteurs mais on pourra alternativement prévoir un nombre inférieur (par exemple deux) ou supérieur (par exemple six ou huit) de secteurs. Les gorges discontinues de la figure 3a permettent notamment d'envisager une indexation de la position angulaire de l'élément de fermeture, sur lequel on prévoira une ou plusieurs languettes 400 de forme complémentaire.

Dans l'exemple de la figure 3b, le pilier intermédiaire 2 comporte trois gorges 290, 292 et 294. Prévoir trois gorges au lieu de deux permet d'augmenter les possibilités de positionnement en hauteur de l'élément de fermeture 4, et de pouvoir mieux s'adapter à l'anatomie du patient. On notera que pour simplifier le pilier 2, on pourra également prévoir une gorge unique. Indépendamment du nombre de gorges prévues, on pourra prévoir que celles-ci soient continues ou discontinues.

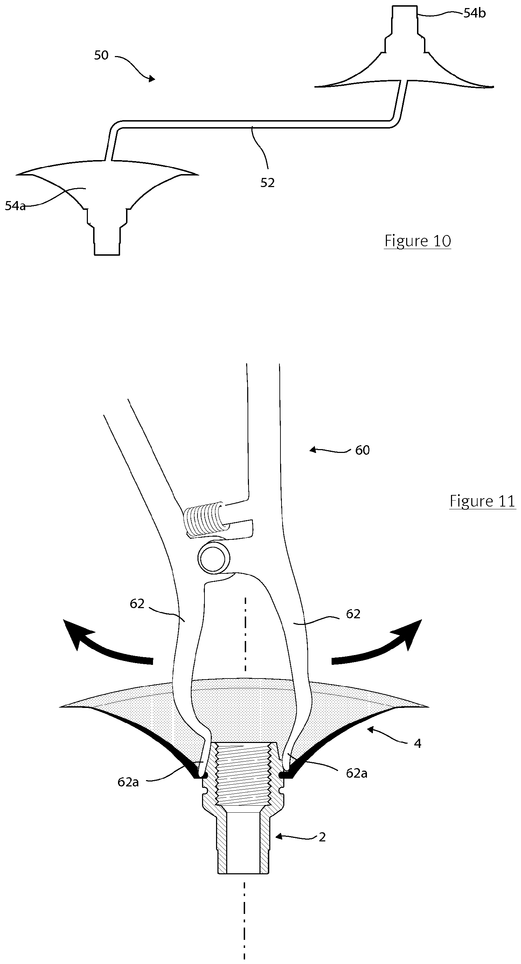

Les figures 10 et 11 montrent des exemples d'outils pouvant aider le praticien lors des interventions mettant en oeuvre un pilier intermédiaire conforme à l'invention.

La figure 10 illustre un exemple d'outil 50 apte à aider le praticien à la sélection d'un élément de fermeture 4 adapté au patient. Dans l'exemple, l'outil comporte une tige de préhension 52 solidaire d'une ou plusieurs formes 54a, 54b représentative(s) du profil de l'ensemble formé par une un élément de fermeture associé à un pilier intermédiaire conforme à l'invention. Ces formes constituent des gabarits permettant au praticien de déterminer, pour un pilier intermédiaire donné, quel élément de fermeture (en fonction de sa taille et de sa forme) associer à ce pilier, et, lorsque le pilier intermédiaire comporte un dispositif de connexion externe 29 à plusieurs niveaux (comme sur les figures 3, 4a et 4b), à quel niveau du pilier fixer l'élément de fermeture. Dans l'exemple, l'outil 50 est représenté avec deux formes 54a, 54b, représentant des éléments de fermeture de hauteurs différentes. Ainsi, grâce à l'outil 50, le praticien peut tester *in situ* quel élément de fermeture correspondra le mieux au site post-extractionnel, sans avoir à ouvrir plusieurs (vrais) éléments de fermeture, ceux-ci étant conditionnés dans des emballages stériles. Avantageusement, l'outil 50 pourra également permettre de sélectionner la forme et/ou la taille du pilier intermédiaire à utiliser. Dans l'exemple de la figure 10, l'outil comporte des formes 54a, 54a qui sont plates et constituent des gabarits en deux dimensions. On pourra bien entendu prévoir des formes constituant des gabarits en trois dimensions.

La figure 11 montre un exemple d'outil 60 de préhension et d'insertion d'un élément de fermeture 4 positionné sur un pilier intermédiaire 2. L'outil 60, qui se présente sous la forme d'une pince d'écartement, comporte dans l'exemple deux bras 60 articulés entre eux, l'extrémité libre 62a de chaque bras étant configurée pour être insérée entre l'élément de fermeture 4 et le pilier intermédiaire 2. Ainsi, l'action de l'outil 60 va permettre d'écarter la partie de fixation 40 de l'élément de fermeture, de manière à sortir les languette 400 des gorges du pilier intermédiaire 2. Avantageusement, l'outil 60 pourra comporter plus de deux bras, par exemple trois ou quatre bras, ces bras étant configurés pour s'écarter selon des directions angulairement équiréparties, afin d'améliorer la répartition des forces d'écartement s'exerçant sur la partie de fixation de l'élément de fermeture.

Avantageusement, le pilier intermédiaire conforme à l'invention sera fourni avec un kit comportant un certain nombre d'éléments de cicatrisation, de formes, de tailles, et de hauteurs différentes.

## Revendications

1. Dispositif de cicatrisation qui comporte :
- un pilier intermédiaire (2) dentaire destiné à être inséré dans un implant (1) dentaire, le pilier intermédiaire (2) comprenant :
o une partie inférieure (20), destinée à être insérée dans un implant (1) ;
o une partie intermédiaire (28), comportant un dispositif externe de connexion (29) d'un élément de fermeture (4) de forme anatomique pour la fermeture du site alvéolaire post-extractionnel, ledit dispositif externe de connexion (29) comportant au moins deux gorges (290, 292) superposées, de forme générale circulaire, ménagées sur la surface externe de la partie intermédiaire (28) du pilier intermédiaire (2)
o une partie supérieure (24), comportant un dispositif de fixation (304) d'un organe (9, 100), tel qu'un pilier d'empreinte (9) ou un pilier prothétique (100)
- et un élément de fermeture (4) anatomique, l'élément de fermeture (4) comportant une partie de fixation (40) destinée à coopérer avec le dispositif externe de connexion (29) du pilier intermédiaire (2), et une partie de fermeture (42) présentant une forme anatomique pour la fermeture du site alvéolaire post-extractionnel.

2. Dispositif de cicatrisation selon la revendication précédente, dans lequel le dispositif externe de connexion (29) est configuré pour permettre la fixation dudit élément de fermeture (4) par coopération de forme, notamment par clipsage avec l'une des gorges du pilier intermédiaire 2.

3. Dispositif de cicatrisation selon l'une des revendications précédentes, comportant au moins trois gorges (290, 292, 294) superposées.

4. Dispositif de cicatrisation selon l'une des revendications 1 à 3, dans lequel les gorges (290, 292, 294) sont continues sur tout le pourtour de la partie intermédiaire (28) du pilier intermédiaire (2).

5. Dispositif de cicatrisation selon l'une des revendications 1 à 3, dans lequel les gorges sont discontinues sur le pourtour de la partie intermédiaire (28) du pilier intermédiaire (2), et comportent une pluralité de secteurs adjacents (290a, 290b ; 290c, 292a, 292b, 292c), par exemple deux, quatre, six ou huit secteurs adjacents.

6. Dispositif de cicatrisation selon l'une des revendications précédentes, **caractérisé en ce que** le pilier intermédiaire (2) est configuré pour que la partie intermédiaire (28) soit, lorsque le pilier intermédiaire (2) est connecté à un implant (1) posé dans la structure osseuse d'un patient, située dans la zone gingivale du site chirurgical, ou partie transmuqueuse.

7. Dispositif de cicatrisation selon l'une des revendications précédentes, dans lequel le dispositif de fixation (304) d'un organe (9, 100) comporte un taraudage (304).

8. Dispositif de cicatrisation selon l'une des revendications précédentes, dans lequel le dispositif de fixation (304) d'un organe (9, 100) est associé à un dispositif d'indexation (240) de la position de l'organe (9, 100) par rapport au pilier intermédiaire (2), et notamment de la position angulaire de l'organe (9, 100).

9. Dispositif de cicatrisation selon l'une des revendications précédentes, comportant une vis (3) permettant la fixation du pilier intermédiaire dans un implant (1), notamment une vis prisonnière.

10. Dispositif de cicatrisation selon la revendication précédente, comportant un alésage (30) traversant, une première portion (300) de l'alésage (30) étant configurée pour permettre le passage de la vis (3), une deuxième portion (302) de l'alésage (30) comportant le dispositif de fixation (304) d'un organe (9, 100).

11. Dispositif de cicatrisation selon les revendications précédentes, dans lequel la partie de fixation (40) de l'élément de fermeture (4) comporte une ou plusieurs languettes (400) déformables élastiquement et configurées pour coopérer avec le dispositif externe de connexion (29) du pilier intermédiaire.

12. Dispositif de cicatrisation selon les revendications précédentes, **caractérisé en ce que** l'élément de fermeture (4) comporte un matériau biocompatible, notamment un polymère biocompatible, tel que le polyétheréthercétone ou le polyuréthane.

13. Dispositif de cicatrisation selon les revendications précédentes, dans lequel la partie de fermeture (42) présente une bordure extérieure (420) de forme dentelée et/ou comportant une surface présentant une rugosité favorisant l'adhésion d'une résine, notamment une résine composite photopolymérisable.

## Patentansprüche

1. Wundverschlussvorrichtung, die Folgendes aufweist:
- Ein zahnmedizinisches Zwischenabutment (2), das dazu bestimmt ist, in ein Zahnimplantat (1) eingesetzt zu werden, wobei das Zwischenabutment (2) Folgendes umfasst:
o Einen unteren Teil (20), der dazu bestimmt, in ein Implantat (1) eingesetzt zu werden;
o einen Zwischenteil (28), der eine externe Verbindungsvorrichtung (29) eines anatomisch geformten Verschlusselements (4) umfasst, um die Stelle der postextraktiven Kavität zu verschließen, wobei die externe Verbindungsvorrichtung (29) mindestens zwei übereinanderliegende Rillen (290, 292) mit einer allgemein kreisförmigen Form aufweist, die auf der Außenfläche des Zwischenteils (28) des Zwischenabutments (2) vorgesehen sind,
o einen oberen Teil (24), der eine Vorrichtung (304) zum Befestigen eines Elements (9, 100), wie eines Abdruck-Abutments (9) oder eines Prothesen-Abutments (100), umfasst,
- und ein anatomisches Verschlusselement (4), wobei das Verschlusselement (4) einen Befestigungsteil (40) umfasst, der dazu bestimmt ist, mit der externen Verbindungsvorrichtung (29) des Zwischenabutments (2) zusammenzuwirken, und einen Verschlussteil (42) mit einer anatomischen Form zum Verschließen der Stelle der postextraktiven Kavität.

2. Wundverschlussvorrichtung nach dem vorhergehenden Anspruch, wobei die externe Verbindungsvorrichtung (29) so ausgelegt ist, dass sie die Befestigung des Verschlusselements (4) durch formschlüssiges Zusammenwirken, insbesondere durch Verklemmen mit einer der Rillen des Zwischenabutments 2, ermöglicht.

3. Wundverschlussvorrichtung nach einem der vorhergehenden Ansprüche, aufweisend mindestens drei übereinanderliegende Rillen (290, 292, 294).

4. Wundverschlussvorrichtung nach einem der Ansprüche 1 bis 3, wobei die Rillen (290, 292, 294) über den gesamten Umfang des Zwischenteils (28) des Zwischenabutments (2) kontinuierlich verlaufen.

5. Wundverschlussvorrichtung nach einem der Ansprüche 1 bis 3, wobei die Rillen am Umfang des Zwischenteils (28) des Zwischenabutments (2) diskontinuierlich verlaufen und eine Vielzahl von benachbarten Sektoren (290a, 290b; 290c, 292a, 292b, 292c) aufweisen, zum Beispiel zwei, vier, sechs oder acht benachbarte Sektoren.

6. Wundverschlussvorrichtung nach einem der vorhergehenden Ansprüche, **dadurch gekennzeichnet, dass** das Zwischenabutment (2) so ausgelegt ist, dass das Zwischenteil (28) sich im Zahnfleischbereich der Operationsstelle oder im transmukosalen Teil befindet, wenn das Zwischenabutment (2) mit einem Implantat (1) verbunden ist, das in die Knochenstruktur eines Patienten eingesetzt ist.

7. Wundverschlussvorrichtung nach einem der vorhergehenden Ansprüche, wobei die Befestigungsvorrichtung (304) eines Elements (9, 100) ein Innengewinde (304) aufweist.

8. Wundverschlussvorrichtung nach einem der vorhergehenden Ansprüche, wobei die Befestigungsvorrichtung (304) eines Elements (9, 100) mit einer Vorrichtung (240) zum Indexieren der Position des Elements (9, 100) relativ zum Zwischenabutment (2), und insbesondere der Winkelposition des Elements (9, 100), zugeordnet ist.

9. Wundverschlussvorrichtung nach einem der vorhergehenden Ansprüche, umfassend eine Schraube (3), die die Befestigung des Zwischenabutments in einem Implantat (1) ermöglicht, insbesondere eine unverlierbare Schraube.

10. Wundverschlussvorrichtung nach dem vorhergehenden Anspruch, umfassend eine Durchgangsbohrung (30), wobei ein erster Abschnitt (300) der Bohrung (30) so ausgelegt ist, dass er einen Durchgang der Schraube (3) ermöglicht, wobei ein zweiter Abschnitt (302) der Bohrung (30) die Befestigungsvorrichtung (304) eines Elements (9, 100) aufweist.

11. Wundverschlussvorrichtung nach den vorhergehenden Ansprüchen, wobei das Befestigungsteil (40) des Verschlusselements (4) eine oder mehrere elastisch verformbare Zungen (400) aufweist, die so ausgelegt sind, dass sie mit der externen Verbindungsvorrichtung (29) des Zwischenabutments zusammenwirken.

12. Wundverschlussvorrichtung nach den vorhergehenden Ansprüchen, **dadurch gekennzeichnet, dass** das Verschlusselement (4) ein biokompatibles Material, insbesondere ein biokompatibles Polymer, wie Polyetheretherketon oder Polyurethan, umfasst.

13. Wundverschlussvorrichtung nach den vorhergehenden Ansprüchen, wobei das Verschlussteil (42) einen Außenrand (420) mit gezackter Form aufweist und/oder eine Oberfläche umfasst, die eine Rauheit aufweist, die die Haftung eines Harzes, insbesondere eines lichthärtenden Verbundharzes, fördert.

## Claims

1. A healing device which includes:
- a dental intermediate pillar (2) to be inserted into a dental implant (1), the intermediate pillar (2) comprising:
o a lower part (20), to be inserted into an implant (1);
o an intermediate part (28), including an outer device (29) for connecting an anatomically-shaped closure element (4) for closing the post-extraction alveolar site, said outer connection device (29) including at least two generally circular-shaped superimposed grooves (290, 292), provided on the outer surface of the intermediate part (28) of the intermediate pillar (2)
o an upper part (24), including a device (304) for attaching a member (9, 100), such as an impression pillar (9) or a prosthetic pillar (100)
- and an anatomical closure element (4), the closure element (4) including an attaching part (40) for cooperating with the outer connection device (29) of the intermediate pillar (2), and a closure part (42) having an anatomical shape for closing the post-extraction alveolar site.

2. The healing device according to the preceding claim, wherein the outer connection device (29) is configured to allow said closure element (4) to be attached by form-fitting, especially by snapping on one of the grooves of the intermediate pillar 2.

3. The healing device according to one of the preceding claims, including at least three superimposed grooves (290, 292, 294).

4. The healing device according to one of claims 1 to 3, wherein the grooves (290, 292, 294) are continuous throughout the perimeter of the intermediate part (28) of the intermediate pillar (2).

5. The healing device according to one of claims 1 to 3, wherein the grooves are discontinuous over the perimeter of the intermediate portion (28) of the intermediate pillar (2), and include a plurality of adjacent sectors (290a, 290b; 290c, 292a, 292b, 292c), for example two, four, six or eight adjacent sectors.

6. The healing device according to one of the preceding claims, **characterised in that** the intermediate pillar (2) is configured so that the intermediate part (28) is, when the intermediate pillar (2) is connected to an implant (1) placed in the bone structure of a patient, located in the gingival zone of the surgical site, or transmucosal part.

7. The healing device according to one of the preceding claims, wherein the device (304) for attaching a member (9, 100) includes a thread (304).

8. The healing device according to one of the preceding claims, wherein the device (304) for attaching a member (9, 100) is associated with a device (240) for indexing the position of the member (9, 100) relative to the intermediate pillar (2), and especially the angular position of the member (9, 100).

9. The healing device according to one of the preceding claims, including a screw (3) for attaching the intermediate pillar in an implant (1), especially a captive screw.

10. The healing device according to the preceding claim, including a through bore (30), a first portion (300) of the bore (30) being configured to allow the screw (3) to pass therethrough, a second portion (302) of the bore (30) including the device (304) for attaching a member (9, 100).

11. The healing device according to the preceding claims, wherein the attaching part (40) of the closure element (4) includes one or more elastically deformable tabs (400) configured to cooperate with the outer connection device (29) of the intermediate pillar.

12. The healing device according to the preceding claims, **characterised in that** the closure element (4) includes a biocompatible material, especially a biocompatible polymer, such as polyetheretherketone or polyurethane.

13. The healing device according to the preceding claims, wherein the closure part (42) has a serrated-shape external edge (420) and/or includes a surface with a roughness promoting adhesion of a resin, especially a photopolymerisable composite resin.
